# EUROPEAN PATENT SPECIFICATION

(11) **EP 3 749 268 B1**
(45) Date of publication and mention of the grant of the patent: **07.01.2026**
(21) Application number: 19706067.6
(22) Date of filing: 29.01.2019
(51) Int. Cl.: A61F 13/36, A61F 13/05, A61F 13/00

(54) **DRESSING FOR DISRUPTION OF DEBRIS AT A TISSUE SITE**
WUNDAUFLAGE ZUR ZERSTÖRUNG VON DEBRIS AN EINER GEWEBESTELLE
PANSEMENT PERMETTANT LA RUPTURE DE DÉBRIS AU NIVEAU D'UN SITE TISSULAIRE

(30) Priority: 05.02.2018 US 201862626334 P
(43) Date of publication of application: 16.12.2020
(73) Proprietor: Solventum Intellectual Properties Company, Maplewood, MN 55144 (US)
(72) Inventor: ALLEN, Diwi L., San Antonio, TX 78255 (US); CARROLL, Christopher Allen, San Antonio, TX 78212 (US); SCHMIDT, Marisa, San Antonio, TX 78250 (US); KHARKAR, Prathamesh, San Antonio, TX 78249 (US)
(74) Representative: Simmons & Simmons
(86) International application number: PCT/US2019/015685
(87) International publication number: WO 2019/152422

(56) References cited:
- WO-A1-2010/080667
- US-A1- 2010 160 876
- US-A1- 2015 320 434

## Description

### TECHNICAL FIELD

The invention set forth in the appended claims relates generally to tissue treatment systems and more particularly, but without limitation, to dressings for application to a tissue site.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound can be washed out with a stream of liquid solution, or a cavity can be washed out using a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

WO2010080667 discloses systems, methods, and apparatuses for delivering reduced pressure to subcutaneous tissue, including a manifold with purging and reduced-pressure lumens, surface features, apertures, and an end cap for fluid coupling.

US2010160876 discloses manifold structures, systems, and methods use longitudinal members and shaped projections to create microstrain at a tissue site, featuring columnar and enlarged members with varying outer diameters.

US2015320434 discloses a method and apparatus for disrupting tissue using a contact layer with through-holes, a sealing member, and a negative-pressure source to form and release nodules.

### BRIEF SUMMARY

The invention is defined by the appended claims in which there is required a tissue interface comprising: a contact layer comprising at least two concentric edges and at least one frangible strut mechanically coupling the concentric edges; wherein the concentric edges and the frangible strut define through-holes in the contact layer having an open area in a range of about 0.25 square centimeters to about 15 square centimeters; wherein the contact layer comprises a spine having a curved or spiral shape that forms the concentric edges.

A selection of optional features is set out in the dependent claims.

### BRIEF DESCRIPTION OF THE DRAWINGS

Figure 1 is a functional block diagram of a therapy system that can provide negative-pressure treatment and instillation treatment in accordance with this specification;
Figure 2 is a graph illustrating additional details of example pressure control modes of the therapy system of Figure 1;
Figure 3 is a graph illustrating additional details that may be associated with another example pressure control mode of the therapy system of Figure 1;
Figure 4 is a perspective view of an unclaimed tissue interface that may be associated with an example of the therapy system of Figure 1;
Figure 5 is a perspective view of an unclaimed tissue interface that may be associated with an example of the therapy system of Figure 1;
Figure 6 is a perspective view of an unclaimed tissue interface that may be associated with an example of the therapy system of Figure 1;
Figure 7 is a perspective view of an unclaimed tissue interface that may be associated with an example of the therapy system of Figure 1;
Figure 8 is a perspective view of an unclaimed tissue interface that may be associated with an example of the therapy system of Figure 1;
Figure 9 is a perspective view of a claimed tissue interface that may be associated with an example of the therapy system of Figure 1; and
Figure 10 is a chart illustrating details that may be associated with an example method of operating the therapy system of Figure 1.

### DESCRIPTION OF EXAMPLES

The following description of examples provides information that enables a person skilled in the art to make and use the subject matter set forth in the appended claims, but may omit certain details already well-known in the art. The following detailed description is, therefore, to be taken as illustrative and not limiting.

The examples may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

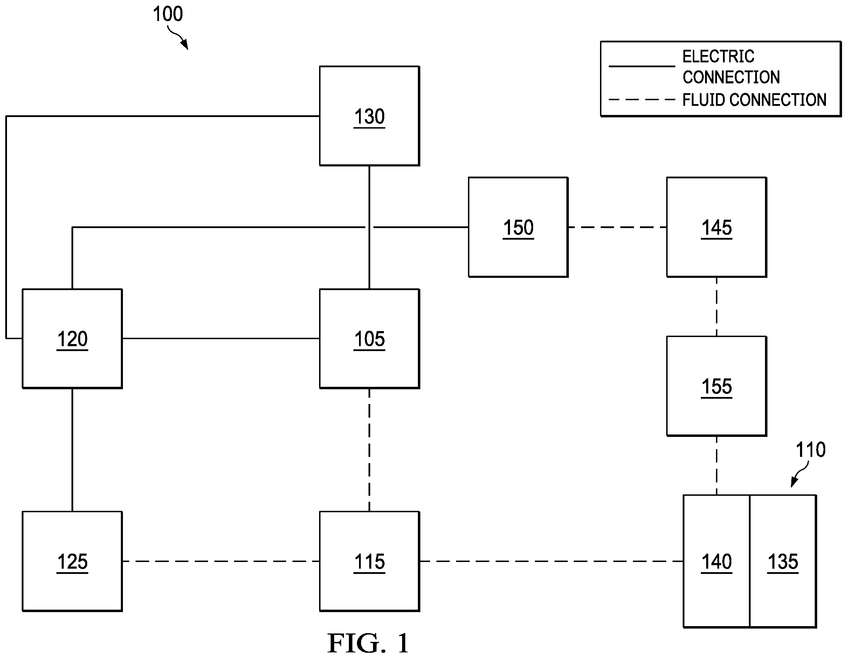

Figure 1 is a simplified functional block diagram of an example of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, a dressing 110, a fluid container, such as a container 115, and a regulator or controller, such as a controller 120, for example. Additionally, the therapy system 100 may include sensors to measure operating parameters and provide feedback signals to the controller 120 indicative of the operating parameters. As illustrated in Figure 1, for example, the therapy system 100 may include a first sensor 125 and a second sensor 130 coupled to the controller 120. As illustrated in the example of Figure 1, the dressing 110 may comprise or consist essentially of a tissue interface 135, a cover 140, or both in some examples.

The therapy system 100 may also include a source of instillation solution. For example, a solution source 145 may be fluidly coupled to the dressing 110, as illustrated in the example of Figure 1. The solution source 145 may be fluidly coupled to a positive-pressure source such as the positive-pressure source 150, a negative-pressure source such as the negative-pressure source 105, or both, in some examples. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 120 may be coupled to the negative-pressure source 105, the positive-pressure source 150, or both, to control dosage of instillation solution to a tissue site. In some examples, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some examples, the negative-pressure source 105 may be combined with the solution source 145, the controller 120 and other components into a therapy unit.

In general, components of the therapy system 100 may be coupled directly or indirectly. For example, the negative-pressure source 105 may be directly coupled to the container 115, and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 120, and may be fluidly coupled to one or more distribution components to provide a fluid path to a tissue site. In some examples, components may also be coupled by virtue of physical proximity, being integral to a single structure, or being formed from the same piece of material.

A distribution component is preferably detachable, and may be disposable, reusable, or recyclable. The dressing 110 and the container 115 are illustrative of distribution components. A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some examples, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

A negative-pressure supply, such as the negative-pressure source 105, may be a reservoir of air at a negative pressure, or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 120, may be a microprocessor or computer programmed to operate one or more components of the therapy system 100, such as the negative-pressure source 105. In some examples, for example, the controller 120 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 135, for example. The controller 120 is also preferably configured to receive one or more input signals, such as a feedback signal, and programmed to modify one or more operating parameters based on the input signals.

Sensors, such as the first sensor 125 and the second sensor 130 are generally known in the art as any apparatus operable to detect or measure a physical phenomenon or property, and generally provide a signal indicative of the phenomenon or property that is detected or measured. For example, the first sensor 125 and the second sensor 130 may be configured to measure one or more operating parameters of the therapy system 100. In some examples, the first sensor 125 may be a transducer configured to measure pressure in a pneumatic pathway and convert the measurement to a signal indicative of the pressure measured. In some examples, for example, the first sensor 125 may be a piezo-resistive strain gauge. The second sensor 130 may optionally measure operating parameters of the negative-pressure source 105, such as a voltage or current, in some examples. Preferably, the signals from the first sensor 125 and the second sensor 130 are suitable as an input signal to the controller 120, but some signal conditioning may be appropriate in some examples. For example, the signal may need to be filtered or amplified before it can be processed by the controller 120. Typically, the signal is an electrical signal, but may be represented in other forms, such as an optical signal.

The tissue interface 135 can be generally adapted to partially or fully contact a tissue site. The tissue interface 135 may take many forms, and may have many sizes, shapes, or thicknesses depending on a variety of factors, such as the type of treatment being implemented or the nature and size of a tissue site. For example, the size and shape of the tissue interface 135 may be adapted to the contours of deep and irregular shaped tissue sites. Moreover, any or all of the surfaces of the tissue interface 135 may have projections or an uneven, course, or jagged profile that can induce strains and stresses on a tissue site, which can promote granulation at the tissue site. The tissue interface 135 may further promote granulation at a tissue site when pressure within the sealed therapeutic environment is reduced. For example, any or all of the surfaces of the tissue interface 135 may have an uneven, coarse, or jagged profile that can induce microstrains and stresses at a tissue site if negative pressure is applied through the tissue interface 135.

In some examples, the cover 140 may provide a bacterial barrier and protection from physical trauma. The cover 140 may also be constructed from a material that can reduce evaporative losses and provide a fluid seal between two components or two environments, such as between a therapeutic environment and a local external environment. The cover 140 may comprise or consist of, for example, an elastomeric film or membrane that can provide a seal adequate to maintain a negative pressure at a tissue site for a given negative-pressure source. The cover 140 may have a high moisture-vapor transmission rate (MVTR) in some applications. For example, the MVTR may be at least 250 grams per square meter per twenty-four hours in some examples, measured using an upright cup technique according to ASTM E96/E96M Upright Cup Method at 38°C and 10% relative humidity (RH). In some examples, an MVTR up to 5,000 grams per square meter per twenty-four hours may provide may provide effective breathability and mechanical properties.

In some examples, the cover 140 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. For example, the cover 140 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some examples, the cover 140 may comprise INSPIRE 2301 having an MVTR (upright cup technique) of 2600 g/m²/24 hours and a thickness of about 30 microns.

An attachment device may be used to attach the cover 140 to an attachment surface, such as undamaged epidermis, a gasket, or another cover. The attachment device may take many forms. For example, an attachment device may be a medically-acceptable, pressure-sensitive adhesive configured to bond the cover 140 to epidermis around a tissue site. In some examples, for example, some or all of the cover 140 may be coated with an adhesive, such as an acrylic adhesive, which may have a coating weight between 25-65 grams per square meter (g.s.m.). Thicker adhesives, or combinations of adhesives, may be applied in some examples to improve the seal and reduce leaks. Other examples of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

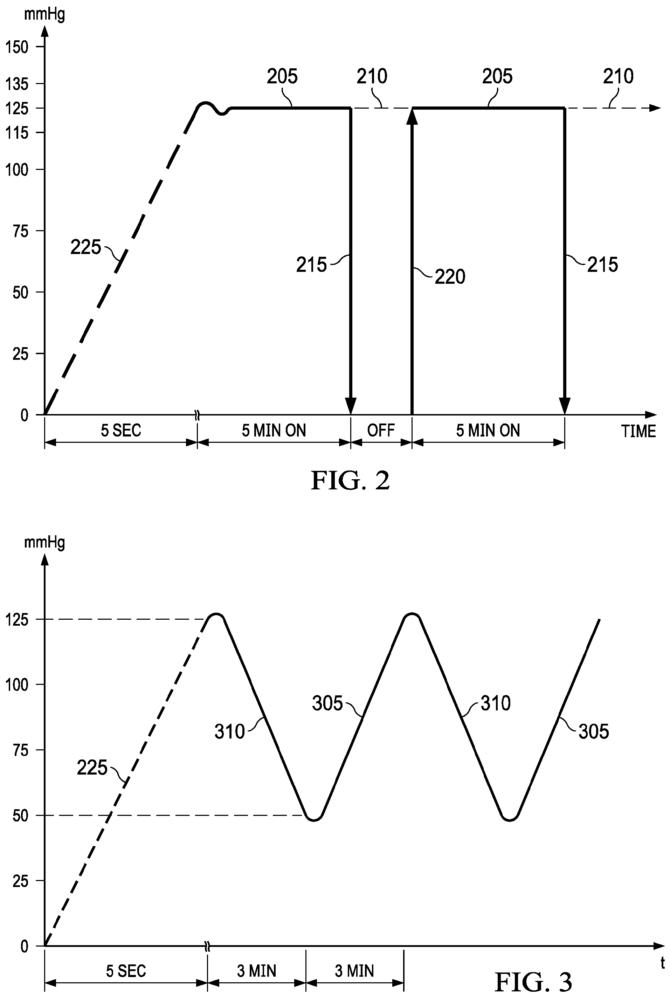

Figure 2 is a graph illustrating additional details of an example control mode that may be associated with some examples of the controller 120. In some examples, the controller 120 may have a continuous pressure mode, in which the negative-pressure source 105 is operated to provide a constant target negative pressure, as indicated by line 205 and line 210, for the duration of treatment or until manually deactivated. Additionally or alternatively, the controller may have an intermittent pressure mode, as illustrated in the example of Figure 2. In Figure 2, the x-axis represents time, and the y-axis represents negative pressure generated by the negative-pressure source 105 over time. In the example of Figure 2, the controller 120 can operate the negative-pressure source 105 to cycle between a target pressure and atmospheric pressure. For example, the target pressure may be set at a value of 125 mmHg, as indicated by line 205, for a specified period of time (e.g., 5 min), followed by a specified period of time (e.g., 2 min) of deactivation, as indicated by the gap between the solid lines 215 and 220. The cycle can be repeated by activating the negative-pressure source 105, as indicated by solid line 220, which can form a square wave pattern between the target pressure and atmospheric pressure.

In some examples, the increase in negative-pressure from ambient pressure to the target pressure may not be instantaneous. For example, the negative-pressure source 105 and the dressing 110 may have an initial rise time, as indicated by the dashed line 225. The initial rise time may vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time as indicated by the solid line 220 may be a value substantially equal to the initial rise time as indicated by the dashed line 225.

Figure 3 is a graph illustrating additional details that may be associated with another example pressure control mode in some examples of the therapy system 100. In Figure 3, the x-axis represents time and the y-axis represents negative pressure generated by the negative-pressure source 105. The target pressure in the example of Figure 3 can vary with time in a dynamic pressure mode. For example, the target pressure may vary in the form of a triangular waveform, varying between a minimum and maximum negative pressure of 50-125 mmHg with a rise time 305 set at a rate of +25 mmHg/min. and a descent time 310 set at -25 mmHg/min, respectively. In other examples of the therapy system 100, the triangular waveform may vary between negative pressure of 25-125 mmHg with a rise time 305 set at a rate of +30 mmHg/min and a descent time 310 set at -30 mmHg/min.

In some examples, the controller 120 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 120, which can vary the target pressure according to a predetermined waveform, such as a triangular waveform, a sine waveform, or a saw-tooth waveform. In some examples, the waveform may be set by an operator as the predetermined or time-varying negative pressure desired for therapy.

In some examples, the tissue interface 135 may be configured for treating a tissue site having debris that may be desirably disrupted. For example, the tissue site may include biofilms, necrotic tissue, lacerated tissue, devitalized tissue, contaminated tissue, damaged tissue, infected tissue, exudate, highly viscous exudate, fibrinous slough, and/or other material that can generally be referred to as debris. Such debris may inhibit the efficacy of tissue treatment and slow the healing of the tissue site.

As an example, during treatment of a tissue site, a biofilm may develop on or in the tissue site. Biofilms may include a microbial infection that can cover a tissue site and impair healing of the tissue site. Biofilms can also lower the effectiveness of topical antibacterial treatments by preventing the topical treatments from reaching the tissue site. The presence of biofilms can increase healing times, reduce the efficacy and efficiency of various treatments, and increase the risk of a more serious infection. Additionally or alternatively, some tissue sites may not heal according to the normal medical protocol and may develop areas of necrotic tissue. Necrotic tissue may include dead tissue resulting from infection, toxins, or trauma that caused the tissue to die faster than the tissue can be removed by the normal body processes that regulate the removal of dead tissue. Sometimes, necrotic tissue may be in the form of slough, which may include a viscous liquid mass of tissue. Generally, slough is produced by bacterial and fungal infections that stimulate an inflammatory response in the tissue. Slough may be a creamy yellow color and may also be referred to as pus. Necrotic tissue may also include eschar. Eschar may be a portion of necrotic tissue that has become dehydrated and hardened. Eschar may be the result of a burn injury, gangrene, ulcers, fungal infections, spider bites, or anthrax. Conventionally, eschar may be generally difficult to move without the use of surgical cutting instruments. In various examples, the debris may cover all or a portion of the tissue site. If the debris is at or in in the tissue site, the tissue site may be treated with various processes to disrupt the debris.

In some examples, the disruption by the tissue interface 135 can include softening of the debris, separation of the debris from desired tissue, such as the subcutaneous tissue, preparation of the debris for removal from the tissue site, and removal of the debris from the tissue site, for example, debridement of the tissue site.

In some examples, the tissue interface 135 may comprise or consist essentially of a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some examples, the fluid path may be reversed or a secondary fluid path may be provided to facilitate delivering fluid such as from a source of instillation solution across a tissue site.

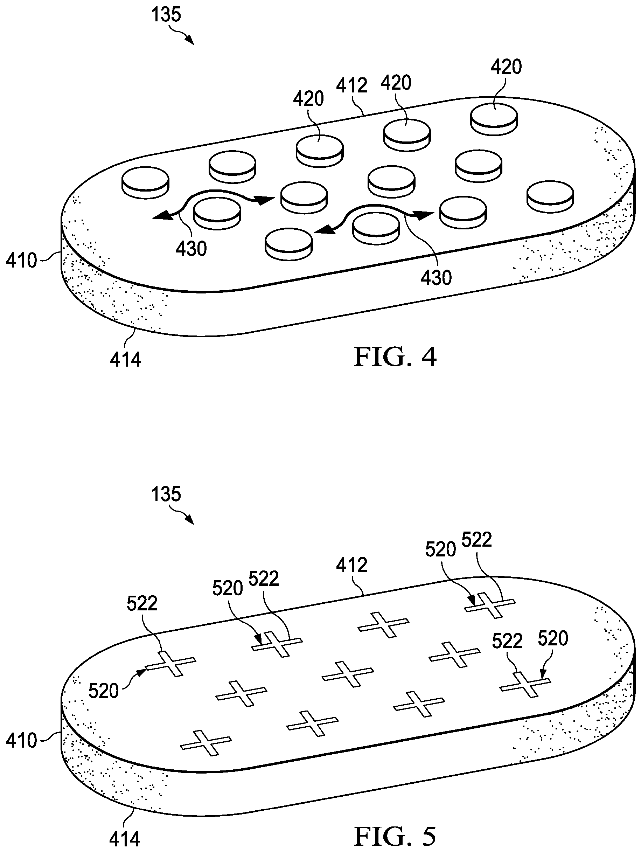

Figure 4 is a schematic diagram illustrating additional details that may be associated with some examples of the tissue interface 135. In the example of Figure 4, the tissue interface 135 generally includes a manifold 410. In some examples, the manifold 410 may include pathways that may be interconnected to improve distribution or collection of fluids across a tissue site. In some examples, the manifold 410 may be a porous material such as foam having interconnected cells or pores. For example, in some examples the manifold 410 may be open-cell foam and may generally include pores, edges, and/or walls adapted to form interconnected fluid channels. Liquids, gels, and other foams may also include or be cured to include apertures and fluid pathways.

The average pore size of foam may vary according to needs of a prescribed therapy. For example, in some examples, the manifold 410 may comprise or consist essentially of foam having pore sizes in a range of 400-600 microns. The tensile strength of the manifold 410 may also vary according to needs of a prescribed therapy. For example, the tensile strength of foam may be increased for instillation of topical treatment solutions. In some examples, the manifold 410 may be reticulated polyurethane foam such as found in GRANUFOAM^{™} dressing or V.A.C. VERAFLO^{™} dressing, both available from Kinetic Concepts, Inc. of San Antonio, Texas.

The manifold 410 may be either hydrophobic or hydrophilic. In some examples in which the manifold 410 may be hydrophilic, the manifold 410 may also wick fluid away from a tissue site, while continuing to distribute negative pressure to the tissue site. The wicking properties of the manifold 410 may draw fluid away from a tissue site by capillary flow or other wicking mechanisms. An example of hydrophilic foam is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity. In some examples, the manifold 410 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones.

The manifold 410 may generally comprise a generally flat structure having two opposite-facing planar surfaces and a depth or thickness orthogonal to the generally planar surfaces. "Planar surfaces" in this context include those surfaces that, although not necessarily perfectly flat, are generally recognized as flat or capable of being laid flat; for example, a generally planar surface may include minor undulations and/or deviations from a single geometric plane. For example, the manifold may include a first surface 412 and a second surface 414.

In some examples, the manifold 410 may comprise a plurality of surface features. For example, in the example of Figure 4, the manifold 410 includes a plurality of surface features 420 disposed on the first surface 412 of the manifold 410. In some examples, the surface features 420 may be configured as projections extending from the first surface 412. In various examples, the surface features 420 may have any suitable shape in a plane generally parallel to the first surface 412. For example, the surface features 420 may be characterized as circles, ovals, triangles, squares, rectangles, pentagons, hexagons, other suitable polygons, cross-hatches, or combinations thereof. The surface features 420 may have an area in a plane generally parallel to the first surface 412 of about 1 mm² to about 5 mm². The surface features 420 may have a height of about 1 mm to about 5 mm. In some examples, the surface features 420 may have a substantially constant area in a cross-sectional plane substantially parallel to the first surface 412. Alternatively, in some examples the surface features 420 may have an area in a cross-sectional plane substantially parallel to the first surface 412 that varies with the height of the surface feature 420. For example, in some examples the surface features 420 may be characterized as pyramidal or conical. In examples where the surface features 420 are at least partially pyramidal or conical, the tip of the surface features 420 may have a surface area of about 0.25 mm² to about 4 mm².

The surface features 420 may be formed from a suitable compressible material. In some examples, the surface features 420 may be formed from foam that is mechanically or chemically compressed, for example, to increase the density of the foam at ambient pressure. Foam that is mechanically or chemically compressed may be referred to as compressed foam. Compressed foam may be characterized with respect to firmness factor (FF). Firmness factor (FF) in this context refers to a ratio of the density of foam in a compressed state relative to the density of the same foam in an uncompressed state. For example, a firmness factor (FF) of 5 may refer to compressed foam exhibiting a density that is five times greater than a density of the same foam in an uncompressed state. Mechanically or chemically compressing foam may reduce a thickness of the foam at ambient pressure when compared to the same foam that has not been compressed. Reducing a thickness of foam by mechanical or chemical compression may increase a density of the foam, which may increase the firmness factor (FF) of the foam. Increasing the firmness factor (FF) of foam may increase a stiffness of the foam in a direction that is parallel to a thickness of the foam. For example, increasing a firmness factor (FF) of the surface features 420 may increase a stiffness of the surface features 420 in a direction that is parallel to the thickness of the surface features 420, for example, in a direction that is generally perpendicular to the first surface 412 of the manifold 410.

The foam material used to form compressed foam may be either hydrophobic or hydrophilic. The foam material used to form compressed foam may also be either reticulated or un-reticulated. The pore size of foam material and the degree to which the foam is compressed may vary according to particular needs of a therapy. For example, in some examples, uncompressed foam may have pore sizes in a range of about 400 microns to about 600 microns. If the same foam is compressed, the pore sizes may be smaller than when the foam is in its uncompressed state.

In some examples, compressed foam may be compressed reticulated polyurethane foam such as found in GRANUFOAM^{™}dressing or V.A.C. VERAFLO^{™} dressing. Compressed reticulated polyurethane foam such as found in GRANUFOAM^{™} may have a density of about 0.03 grams per centimeter³ (g/cm³) in its uncompressed state. If the compressed reticulated polyurethane foam such as found in GRANUFOAM^{™} is compressed to have a firmness factor (FF) of 5, the resultant foam may be compressed until the density of the foam is about 0.15g/cm³. Reticulated polyurethane foam such as found V.A.C. VERAFLO^{™} dressing may also be compressed to form a compressed foam having a firmness factor (FF) up to 5.

In some examples, compressed foam may comprise or be felted foam. Generally, felted foam undergoes a thermoforming process to permanently compress the foam to increase the density of the foam. Felted foam may also be characterized with respect to other felted foams or compressed foams by firmness factor. Generally compressed foam or felted foam may have a firmness factor greater than 1.

Generally, if compressed foam is subj ected to negative pressure, the compressed foam exhibits less deformation than a similar uncompressed foam. For example, if the surface features 420 formed of compressed foam, the thickness of the surface features 420 may deform less than if the surface features 420 is formed of a comparable uncompressed foam. The decrease in deformation may result from the increased stiffness as reflected by the firmness factor (FF). For example, if subjected to the stress of negative pressure, the surface features 420 formed of compressed foam may flatten less than otherwise similar surface features 420 formed from uncompressed foam. Consequently, if negative pressure is applied to the surface features 420, the stiffness of the compressed foam forming the surface features 420 in the direction parallel to the thickness of the surface features 420 allows the surface features 420 to be more compliant or compressible in other directions, for example, a direction perpendicular to the thickness of the surface features.

In some examples, the surface features 420 may be characterized as exhibiting a density that is at least the density of the manifold 410 or as exhibiting a density that is greater than the density of the manifold 410. For example, in some examples the surface features 420 may be characterized as exhibiting a density that is at least about 120%, 140%, 160%, 180%, 200%, 220%, 240% 260%, 280%, 300%, 350%, 400%, 450%, or 500% the density of the manifold 410.

The surface features 420 may be disposed on the first surface 412 of the manifold 410 in any suitable fashion, for example, in accordance with the needs of a particular therapy. For example, in various examples, the surface features 420 may be disposed on the first surface uniformly or may be relatively more concentrated in certain regions of the first surface 412 and relatively less concentrated in other regions of the first surface 412. In some examples, the surface features 420 may be disposed on the first surface 412 such that, when the tissue interface 135 is positioned with the surface features 420 in contact with a tissue site, the surface features 420 will yield void spaces laterally between adjacent surface features 420. In some examples, the void spaces between the surface features 420 may form interconnected fluid pathways 430.

In some examples, upon positioning the tissue interface with respect to a tissue site and the application of negative pressure to the tissue interface 135, the surface features 420 may be effective to create deformation, stresses, or strains at the tissue site that may have the effect of disrupting debris. Additionally or alternatively, the application of negative pressure to the tissue interface 135 may be effective to cause debris to be drawn into the pores or other open spaces of the manifold 410 such that the debris may be removed from the tissue site.

Additionally or alternatively, in some examples the surface features may be integrated within the manifold 410. For example, Figure 5 illustrates an example of a tissue interface 135 including a manifold 410 having surface features 520 that may be disposed within the manifold 410. In some examples, the surface features 520 may have an outer surface 522 that is substantially coplanar with the first surface 412 of the manifold. The surface features 520 may have any suitable shape in a plane generally parallel to the first surface 412, for example, circles, ovals, triangles, squares, rectangles, pentagons, hexagons, other suitable polygons, cross-hatches, or combinations thereof.

In some examples, upon positioning the tissue interface with respect to a tissue site and the application of negative pressure to the tissue interface 135, the manifold 410 may compress or deflect more relative to the surface features 520, for example, so as to form channels, grooves, or void spaces between adjacent surface features. The resulting pattern may allow the surface features 520 to create deformation, stresses, or strains at the tissue site that may have the effect of disrupting debris. Additionally or alternatively, the application of negative pressure to the tissue interface 135 may be effective to cause debris to be drawn into the pores or other open spaces of the manifold 410 such that the debris may be removed from the tissue site.

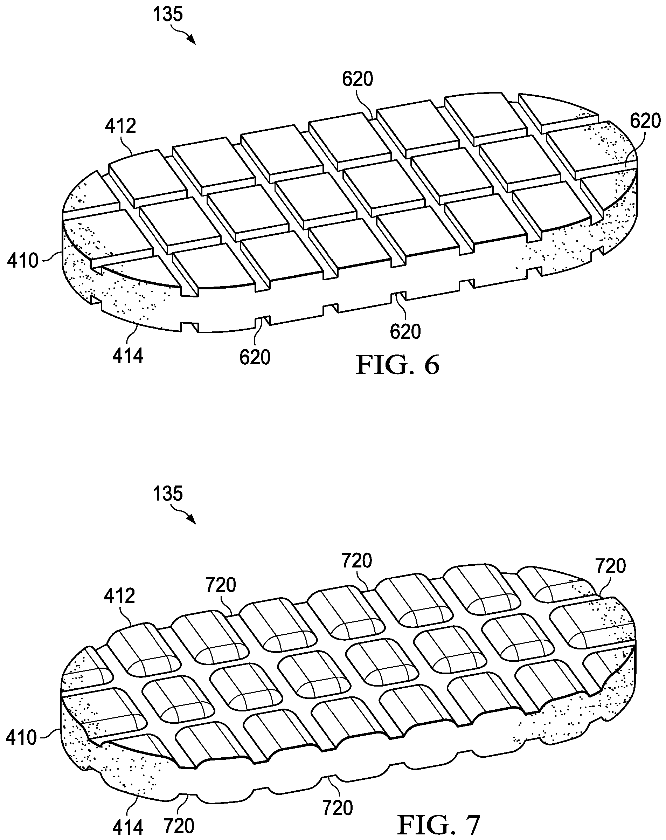

Additionally or alternatively, in some examples the surface features may be characterized as grooves, furrows, hollows, passageways, or channels disposed beneath the first surface 412, the second surface 414, or both. For example, Figure 6 illustrates an example of a tissue interface 135 including a manifold 410 having surface features configured as channels 620 disposed within the first surface 412 and the second surface 414 of the manifold. The channels 620 may have any suitable size and configuration. For example, in Figure 6, the channels 620 may be characterized as having straight side-walls and a square or rectangular cross-section. In various examples, the channels 620 may have any suitable spacing and may intersect at a suitable angle. For example, in Figure 6 the channels 620 may generally form a grid pattern.

Figure 7 illustrates another example of a tissue interface 135 including a manifold 410 having surface features configured as channels 720 disposed within the first surface 412 and the second surface 414 of the manifold. In the example of Figure 7, the channels 720 may be characterized as having a curved side-walls.

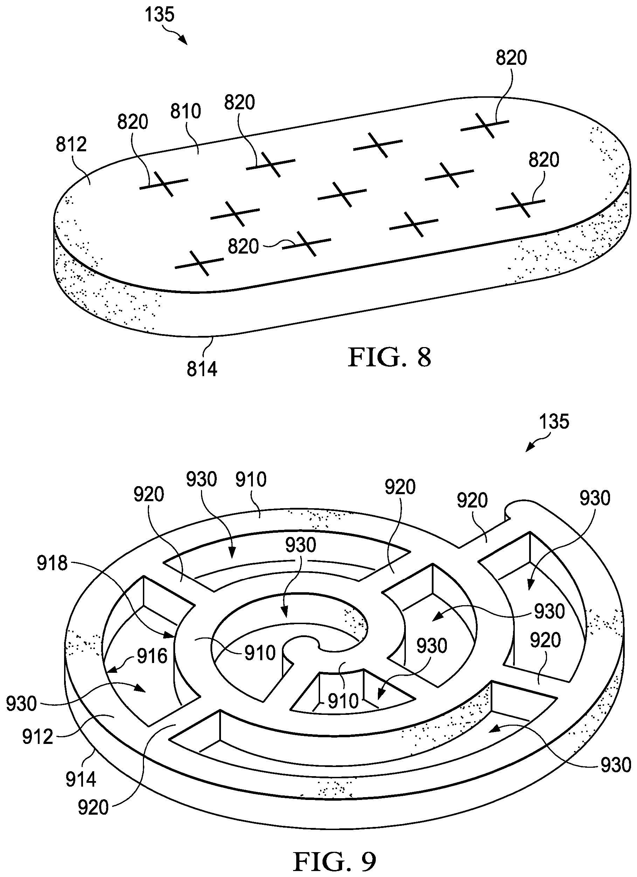

In another example, the tissue interface 135 may comprise a manifold including a plurality of slits. For example, Figure 8 illustrates an example of a tissue interface 135 including a manifold 810 having a plurality of slits 820 extending at least partially through the manifold 810. The term "slit" in this context broadly refers to a discontinuity in the manifold 810. Generally, no material is removed when the slit 820 is formed in the manifold 810. The manifold 810 may generally define two opposite-facing planar surfaces, for example, a first surface 812 and a second surface 814.

In some examples, the slits 820 may extend partially through the thickness of the manifold 810, for example, from the first surface 812 toward the second surface 814 or from the second surface 814 toward the first surface 812. In some examples, the slits 820 may extend fully through the thickness of the manifold 810, for example, between the first surface 812 and the second surface 814.

In some examples, the slits 820 may be disposed uniformly across the first surface 812 and/or the second surface 814. In some examples, the slits 820 may be relatively more concentrated in certain regions of the first surface 812 and/or the second surface 814 and relatively less concentrated in other regions of the first surface 812 and/or the second surface 814. In some examples, the slits 820 may be disposed in the manifold 810 in a cross-hatched pattern.

In some examples, the manifold 810 may be formed from or comprise foam, such open-cell foam and/or compressed foam. In some examples, the manifold 810 may comprise a combination of materials, for example, a compressed foam layer and an open-cell foam layer.

In some examples, the slits 820 within the manifold 810 may be configured such that the application of negative pressure to the manifold 810 may be effective to cause deflection of at least a portion of the manifold 810, for example, near the slits 820. For example, intermittent application of negative pressure to the manifold 810 may cause the slits 820 to exhibit alternating opening and closing motion. In some examples, the deflection of at least a portion of the manifold 810 due to the presence of the slits 820 may be effective to create deformation, stresses, or strains at the tissue site that may have the effect of disrupting debris. Additionally or alternatively, the application of negative pressure to the tissue interface 135 may be effective to cause debris to be drawn into the pores or other open spaces of the manifold 810 such that the debris may be removed from the tissue site.

In another example, the tissue interface 135 may be generally spirally-shaped. For example, Figure 9 illustrates an example of a tissue interface 135 including a spine 910 and a plurality of struts 920 mechanically coupling the edges of the spine 910. The spine 910 and the plurality of struts 920 may cooperatively define two opposite-facing planar surfaces, for example, a first surface 912 and a second surface 914, and a thickness extending between the first surface 912 and the second surface 914. The spine 910 and struts 920 may have a suitable thickness, for example, about 2 mm to about 20 mm.

In various examples, the spine 910 and the struts 920 may comprise or be formed from foam such as open-cell foam compressed foam, film, silicone, thermoplastic elastomer, electrospun textile, or combinations thereof. In some examples, the spine 910 and the struts 920 may comprise or be formed from the same or a similar material. In other examples, the spine 910 and the struts may comprise or be formed from different or dissimilar materials, according to the needs of a particular therapy. The spine 910 and struts 920 may be hydrophilic or hydrophobic, according to the needs or a particular therapy.

The spine 910 is generally spirally-shaped, for example, such that the spine 910 includes generally concentric edges. For example, the spine 910 may include a concave surface 916, which may be inward-facing, and a convex surface 918, which may be outward-facing. The spine 910 may have a suitable width extending between the concave surface 916 and the convex surface 918, for example, about 1 cm to about 5 cm. In some examples, the spine 910 may have a substantially constant width. In other examples, the spine 910 may be generally tapered toward an end, for example, comprising a spire. The spine 910 may extend spirally for a suitable number of rotations, for example, about 1, 1 ½, 2, 2 ½, 3, 3 ½, 4, 4 ½, 5, 5 ½, 6, 6 ½, 7, 7 ½, 8, or more rotations, depending upon the needs of a particular therapy. The spine may also have a suitable length, for example, about 10 cm to about 100 cm.

In some examples, the struts 920 may mechanically couple the adjacent rotations of the spine 910. For example, the struts 920 may mechanically couple the convex surface 918 of the spine 910 to concave surface 916 of the spine 910 at a location where the convex surface 918 and the concave surface 916 are proximate as a result of the spiral configuration of the spine 910.

The struts 920 are characterized as frangible. For example, the struts 920 may be configured such that the struts can be separated by a user, such as by cutting or tearing. In some examples, the struts 920 may have a tear-strength of about 1 N to about 15 N. The frangible nature of the struts 920 may allow the tissue interface 135 to be sized by a user to a particular tissue site. The struts 920 may have a suitable width, for example, about 0.5 cm to about 2.5 cm. In some examples, the struts 920 may have a substantially constant width. In other examples, the struts 920 may be generally tapered toward, for example, comprising a spire.

The spine 910 and the struts 920 cooperatively define a plurality of open spaces 930. In various examples, the open spaces 930 may have any suitable size or shape. For example, the open spaces 930 may be wedge-shaped or pie-shaped. The open spaces 930 may have an area of about 0.25 cm² to about 15 cm². In some examples, the ratio of the total area of the open spaces 930 to about the total area of the spine 910 and the struts 920 may be from about 4:1 to about 1:4.

In some examples, upon positioning the tissue interface with respect to a tissue site and the application of negative pressure to the tissue interface 135, the combination of the spine 910, the struts 920, and the open spaces 930 may create deformation, stresses, or strains at the tissue site that may have the effect of disrupting debris. Additionally or alternatively, the application of negative pressure to the tissue interface 135 may be effective to cause debris to be drawn into the open spaces 930 such that the debris may be removed from the tissue site.

In operation, the tissue interface 135 may be placed within, over, on, or otherwise proximate to a tissue site. If the tissue site is a wound, for example, the tissue interface 135 may partially or completely fill the wound, or may be placed over the wound. The cover 140 may be placed over the tissue interface 135 and sealed to an attachment surface near a tissue site. For example, the cover 140 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

In some examples, the application of negative pressure to the tissue interface 135 may be effective to disrupt debris present at the tissue site. For example, the various examples of the tissue interface 135 may be effective, upon the application of negative pressure to the tissue interface 135, to create deformation, stresses, or strains at the tissue site that may have the effect of disrupting debris. Negative pressure applied across the tissue site through the tissue interface 135 in the sealed therapeutic environment can induce macro-strain and micro-strain in the tissue site. Additionally or alternatively, the various examples may be effective to remove debris from the tissue site. For example, the application of negative pressure to the various examples of the tissue interface 135 may cause debris to be drawn into pores, apertures, or open spaces of the tissue interface 135 such that the debris may be removed from the tissue site. Negative pressure can also remove exudate and other fluid from a tissue site, which can be collected in container 115.

In some examples, the controller 120 may receive and process data from one or more sensors, such as the first sensor 125. The controller 120 may also control the operation of one or more components of the therapy system 100 to manage the pressure delivered to the tissue interface 135. In some examples, controller 120 may include an input for receiving a desired target pressure, and may be programmed for processing data relating to the setting and inputting of the target pressure to be applied to the tissue interface 135. In some examples, the target pressure may be a fixed pressure value set by an operator as the target negative pressure desired for therapy at a tissue site and then provided as input to the controller 120. The target pressure may vary from tissue site to tissue site based on the type of tissue forming a tissue site, the type of injury or wound (if any), the medical condition of the patient, and the preference of the attending physician. After selecting a desired target pressure, the controller 120 can operate the negative-pressure source 105 in one or more control modes based on the target pressure, and may receive feedback from one or more sensors to maintain the target pressure at the tissue interface 135.

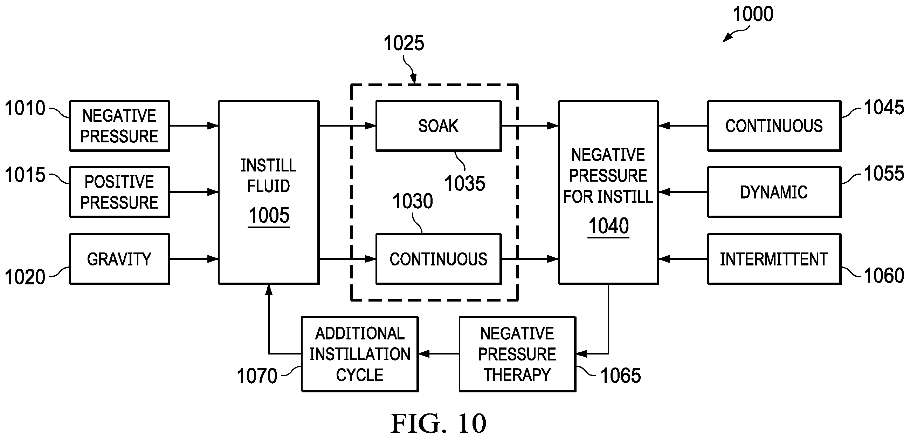

Figure 10 is a chart illustrating details that may be associated with an example method 1000 of operating the therapy system 100 to provide negative-pressure treatment and instillation treatment to the tissue interface 135. In some examples, the controller 120 may receive and process data, such as data related to instillation solution provided to the tissue interface 135. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 120 may also control the operation of one or more components of the therapy system 100 to instill solution, as indicated at 1005. For example, the controller 120 may manage fluid distributed from the solution source 145 to the tissue interface 135. In some examples, fluid may be instilled to a tissue site by applying a negative pressure from the negative-pressure source 105 to reduce the pressure at the tissue site, drawing solution into the tissue interface 135, as indicated at 1010. In some examples, solution may be instilled to a tissue site by applying a positive pressure from the positive-pressure source 150 to move solution from the solution source 145 to the tissue interface 135, as indicated at 1015. Additionally or alternatively, the solution source 145 may be elevated to a height sufficient to allow gravity to move solution into the tissue interface 135, as indicated at 1020.

The controller 120 may also control the fluid dynamics of instillation at 1025 by providing a continuous flow of solution at 1030 or an intermittent flow of solution at 1035. Negative pressure may be applied to provide either continuous flow or intermittent flow of solution at 1040. The application of negative pressure may be implemented to provide a continuous pressure mode of operation at 1045 to achieve a continuous flow rate of instillation solution through the tissue interface 135, or may provide a dynamic pressure mode of operation at 1055 to vary the flow rate of instillation solution through the tissue interface 135. Alternatively, the application of negative pressure may be implemented to provide an intermittent mode of operation at 1060 to allow instillation solution to dwell at the tissue interface 135. In an intermittent mode, a specific fill volume and dwell time may be provided depending, for example, on the type of tissue site being treated and the type of dressing being utilized. After or during instillation of solution, negative-pressure treatment may be applied at 1065. The controller 120 may be utilized to select a mode of operation and the duration of the negative pressure treatment before commencing another instillation cycle at 1070 by instilling more solution at 1005.

The systems, apparatuses, and methods described herein may provide significant advantages. For example, by disrupting debris at the tissue site, removing debris from the tissue site, or both, the tissue interface 135 may be effective to encourage granulation at the tissue site and stimulate wound healing
While shown in a few illustrative examples, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the container 115, or both may be eliminated or separated from other components for manufacture or sale. In other example configurations, the controller 120 may also be manufactured, configured, assembled, or sold independently of other components.

## Claims

1. A tissue interface comprising:
a contact layer comprising at least two concentric edges (916, 918) and at least one frangible strut (920) mechanically coupling the concentric edges (916, 918);
wherein the concentric edges (916, 918) and the frangible strut (920) define through-holes (930) in the contact layer having an open area in a range of about 0.25 square centimeters to about 15 square centimeters;
wherein the contact layer comprises a spine (910) having a curved or spiral shape that forms the concentric edges (916, 918).

2. The tissue interface of claim 1, the spine (910) having a spiral shape with at least one full turn and spires that form the concentric edges (916, 918).

3. The tissue interface of claim 1, wherein:
the spine (910) has a curved shape; and
the spine (910) has a width in a range of about 2 millimeters to about 20 centimeters.

4. The tissue interface of claim 1, wherein:
the spine (910) has a curved shape; and
the spine (910) has a width in a range of about 1 centimeter to about 5 centimeters.

5. The tissue interface of claim 1, wherein:
the spine (910) has a curved shape;
the spine (910) has a thickness in a range of about 2 millimeters to about 20 centimeters; and
the spine (910) has a width in a range of about 1 centimeter to about 5 centimeters.

6. The tissue interface of claim 1, wherein the contact layer is formed from a compressible material.

7. The tissue interface of claim 1, wherein the contact layer is formed from foam, film, silicone, thermoplastic elastomer, or electrospun textile.

8. The tissue interface of claim 1, wherein the contact layer is formed from felted foam.

## Patentansprüche

1. Eine Gewebeschnittstelle, aufweisend:
eine Kontaktschicht, aufweisend mindestens zwei konzentrische Kanten (916, 918) und mindestens eine zerbrechliche Strebe (920), die die konzentrischen Kanten (916, 918) mechanisch koppelt;
wobei die konzentrischen Kanten (916, 918) und die zerbrechliche Strebe (920) Durchgangslöcher (930) in der Kontaktschicht definieren, die eine offene Fläche in einem Bereich von etwa 0,25 Quadratzentimetern bis etwa 15 Quadratzentimetern haben;
wobei die Kontaktschicht einen Rücken (910) aufweist, der eine gekrümmte oder spiralförmige Form hat, der die konzentrischen Kanten (916, 918) bildet.

2. Die Gewebeschnittstelle nach Anspruch 1, wobei der Rücken (910) eine Spiralform mit mindestens einer vollen Windung und Spitzen hat, die die konzentrischen Kanten (916, 918) bilden.

3. Die Gewebeschnittstelle nach Anspruch 1, wobei:
der Rücken (910) eine gekrümmte Form hat; und
der Rücken (910) eine Breite in einem Bereich von etwa 2 Millimetern bis etwa 20 Zentimetern hat.

4. Die Gewebeschnittstelle nach Anspruch 1, wobei:
der Rücken (910) eine gekrümmte Form hat; und
der Rücken (910) eine Breite in einem Bereich von etwa 1 Zentimeter bis etwa 5 Zentimetern hat.

5. Die Gewebeschnittstelle nach Anspruch 1, wobei:
der Rücken (910) eine gekrümmte Form hat;
der Rücken (910) eine Dicke in einem Bereich von etwa 2 Millimetern bis etwa 20 Zentimetern hat; und
der Rücken (910) eine Breite in einem Bereich von etwa 1 Zentimeter bis etwa 5 Zentimetern hat.

6. Die Gewebeschnittstelle nach Anspruch 1, wobei die Kontaktschicht aus einem komprimierbaren Material gebildet ist.

7. Die Gewebeschnittstelle nach Anspruch 1, wobei die Kontaktschicht aus Schaum, Folie, Silikon, thermoplastischem Elastomer oder elektrogesponnenem Textil gebildet ist.

8. Die Gewebeschnittstelle nach Anspruch 1, wobei die Kontaktschicht aus Filzschaum gebildet ist.

## Revendications

1. Interface tissulaire comprenant :
une couche de contact comprenant au moins deux bords concentriques (916, 918) et au moins une entretoise frangible (920) accouplant mécaniquement les bords concentriques (916, 918) ;
dans laquelle les bords concentriques (916, 918) et l'entretoise frangible (920) définissent des trous traversants (930) dans la couche de contact ayant une zone ouverte comprise entre environ 0,25 centimètre carré et environ 15 centimètres carrés ;
dans laquelle la couche de contact comprend une colonne vertébrale (910) ayant une forme incurvée ou en spirale qui forme les bords concentriques (916, 918).

2. Interface tissulaire selon la revendication 1, la colonne vertébrale (910) ayant une forme en spirale avec au moins un tour complet et des spires qui forment les bords concentriques (916, 918).

3. Interface tissulaire selon la revendication 1, dans laquelle :
la colonne vertébrale (910) a une forme incurvée ; et
la colonne vertébrale (910) a une largeur dans une plage allant d'environ 2 millimètres à environ 20 centimètres.

4. Interface tissulaire selon la revendication 1, dans laquelle :
la colonne vertébrale (910) a une forme incurvée ; et
la colonne vertébrale (910) a une largeur dans une plage allant d'environ 1 centimètre à environ 5 centimètres.

5. Interface tissulaire selon la revendication 1, dans laquelle :
la colonne vertébrale (910) a une forme incurvée ;
la colonne vertébrale (910) a une épaisseur dans une plage allant d'environ 2 millimètres à environ 20 centimètres ; et
la colonne vertébrale (910) a une largeur dans une plage allant d'environ 1 centimètre à environ 5 centimètres.

6. Interface tissulaire selon la revendication 1, dans laquelle la couche de contact est formée d'un matériau compressible.

7. Interface tissulaire selon la revendication 1, dans laquelle la couche de contact est formée à partir de mousse, film, silicone, élastomère thermoplastique ou textile électrofilé.

8. Interface tissulaire selon la revendication 1, dans laquelle la couche de contact est formée à partir d'une mousse feutrée.
